# EUROPEAN PATENT SPECIFICATION

(11) **EP 1 903 338 B1**
(45) Date of publication and mention of the grant of the patent: **21.08.2013**
(21) Application number: 07105732.7
(22) Date of filing: 05.04.2007
(51) Int. Cl.: G01N 33/543, C12N 15/10, B01J 20/00, B01L 3/00, C12N 13/00

(54) **Method and Apparatus for Isolating and Purifying Nucleic Acid by Single Bead**
Verfahren und Vorrichtung zur Isolation und Reinigung von Nukleinsäure durch einzelne Kügelchen
Procédé et appareil pour l'isolation et la purification d'acide nucléiques par bille unique

(30) Priority: 25.09.2006 KR 20060092921
(43) Date of publication of application: 26.03.2008
(73) Proprietor: Samsung Electronics Co., Ltd., Suwon-si, Gyeonggi-do, 443-742 (KR)
(72) Inventor: Yoo, Chang-eun, Gyeonggi-do (KR); Jeong, Sung-young, Gyeonggi-do (KR); Kim, Young-rok, Gyeonggi-do (KR)
(74) Representative: Grünecker, Kinkeldey, Stockmair & Schwanhäusser

(56) References cited:
- EP-A- 1 715 039
- EP-A- 1 724 016
- EP-A- 1 842 914
- EP-A1- 1 870 449
- EP-A1- 1 876 231
- EP-A2- 1 650 297
- EP-A2- 1 655 366
- EP-A2- 1 662 008
- WO-A-02/48164
- WO-A-98/51693
- WO-A-2004/055213
- WO-A2-2007/149136
- POMPE T ET AL: "Maleic anhydride copolymers - A versatile platform for molecular biosurface engineering" BIOMACROMOLECULES, ACS, WASHINGTON, DC, US, vol. 4, no. 4, 2003, pages 1072-1079, XP002293090 ISSN: 1525-7797
- VEYRET ET AL: "Magnetic colloids for the generic capture of viruses" ANALYTICAL BIOCHEMISTRY, ACADEMIC PRESS, NEW YORK, NY, US, vol. 346, no. 1, 1 November 2005 (2005-11-01), pages 59-68, XP005109905 ISSN: 0003-2697
- ROBIN HUI LIU; ET AL: "SELF-CONTAINED, FULLY INTEGRATED BIOCHIP FOR SAMPLE PREPARATION, POLYMERASE CHAIN REACTION AMPLIFICATION, AND DNA MICROARRAY DETECTION" ANALYTICAL CHEMISTRY, AMERICAN CHEMICAL SOCIETY. COLUMBUS, US, vol. 76, no. 7, 1 April 2004 (2004-04-01), pages 1824-1831, XP001196720 ISSN: 0003-2700
- LEE JEONG-GUN; ET AL: "Microchip-based one step DNA extraction and real-time PCR in one chamber for rapid pathogen identification" LAB ON A CHIP, ROYAL SOCIETY OF CHEMISTRY, CAMBRIDGE, GB, vol. 6, no. 7, 1 January 2006 (2006-01-01) , pages 886-895, XP002412028 ISSN: 1473-0197

<110> Samsung Electronics Co., Ltd.
<120> Method and apparatus for isolating and purifying nucleic acid by single bead
<130> EP49907FZ163pau
<140> notyetassigned
   <141> notyetassigned
<150> Korean Patent Application No. 10-2006-0092921
   <151> 2006-09-25
<160> 2
<170> KopatentIn 1.71
<210> 1
   <211> 20
   <212> DNA
   <213> Artificial Sequence
<220>
   <223> forward primer
<400> 1
   yccakactcc tacgggaggc 20
<210> 2
   <211> 22
   <212> DNA
   <213> Artificial Sequence
<220>
   <223> reverse primer
<400> 2
   gtattaccgc ttctgctggc ac 22

## Description

### BACKGROUND OF THE INVENTION

### 1. Field of the Invention

The present invention relates to a method and apparatus for isolating and purifying nucleic acid.

### 2. Description of the Related Art

In general, to extract DNA from a clinical sample, processes of concentrating target cells in a sample, disrupting the concentrated cells, isolating DNA after cell disruption and purifying the isolated DNA have to be performed. To perform such processes on a solid support, surfaces having different chemical functional groups introduced according to each operation have to be used. In general, a surface used for cell concentration and a surface used for DNA purification have different functional groups. Therefore, in order to use such a solid support comprising surfaces with different functional groups according to each operation in a lab-on-a-chip, separate chambers having surfaces appropriate to each operation should be manufactured, resulting in a complication of the system. In addition, fluids that are flowed into each chamber should be adjusted, and thus a plurality of pumps and valves are required. Therefore, there is a need for a method of extracting genes from cells in a clinical sample using a single surface.

US Patent No. 6,617,105 discloses a method of isolating nucleic acid from a sample containing cells, the method comprising: binding cells in the sample to a solid support coated with a cell binding moiety; disrupting the isolated cell; binding nucleic acid released from the disrupted cells to the solid support; and recovering the nucleic acid from the solid support. However, in US Patent No. 6,617,105, chaotropic salts or detergents are used for cell disruption, and a Boom method (US Patent No. 5,234,089) is used for nucleic acid purification.

WO 02/48164 A describes materials for extracting nucleic acid using charged switching methods, said materials comprising an ionizable group. The ionizable groups may be immobilized on a solid support, e.g. a magnetic particle.

WO 2004/055213 A provides charge switching polyfunctional reagents for reversibly binding to target substances, e.g. nucleic acids or cells. The polyfunctional reagents may be attached to a solid phase, such as magnetic beads.

POMPE et al. (2003) BIOMACROMOLECULES 4, 1072-1079, relates to thin polymaleic coatings for the functional modulation of immobilized bioactive molecules at solid/liquid interfaces. The approach is based on equivalently attached alternating maleic acid anhydride copolymers with a variety of co-monomers and extended through conversion of the anhydride moieties.

VEYRET et al. (2005) ANAL BIOCHEM. 346, 59-68, discloses magnetic colloids for the generic capture of viruses. The magnetic colloids are coated with a layer of polyethyleneimine followed by absorption of a polymaleic anhydride ether onto the polyethyleneimine for providing an ionic magnetic particles that can be used to capture viruses.

EP 1 715 039 discloses a method of isolating a nucleic acid using a bifunctional material containing an amino group and a carboxyl group, whereby a pH change is used to bind and release nucleic acids to the bifunctional material.

EP 1 724 016 refers to a pH dependent ion exchange material that is capable of selectively binding to nucleic acids, as well as solid substrates having said material immobilized on the surface.

EP 1 842 914 discloses a method and an apparatus for disrupting cells and purifying nucleic acid using a single chamber, whereby a solid support onto which a lysis enhancement metal oxide and a binding metal oxide, e.g. Fe₂O₃ and Al₂O₃, respectively, are deposited.

Therefore, the inventors of the present invention have earnestly studied to solve the problems of the prior art, and found that by binding a compound having a hydrophobic moiety for cell separation and a pH dependent charge switching moiety for nucleic acid purification to the surface of a single bead, cells or viruses can be isolated and nucleic acid can be efficiently isolated and purified from the isolated cells or viruses by using a single surface and aqueous buffers, thus completing the present invention.

### SUMMARY OF THE INVENTION

The present invention provides a method of isolating and purifying nucleic acid from a cell or virus according to claim 1 using single beads to which a compound having a hydrophobic moiety for cell separation and a pH dependent charge switching moiety for nucleic acid purification is bound, wherein cell concentration, cell disruption and nucleic acid purification can be efficiently performed on the single beads.

The present invention also provides an apparatus for isolating and purifying nucleic acid from a cell or virus according to claim 13 using single beads to which a compound having a hydrophobic moiety for cell separation and a pH dependent charge switching moiety for nucleic acid purification is bound, wherein cell concentration, cell disruption and nucleic acid purification can be efficiently performed on the single beads.

The present invention also provides a lab-on-a-chip (LOC) including the apparatus.

The present invention also provides a bead for isolating and purifying nucleic acid from a cell or virus, wherein a compound represented by Formula 1 is bound to the surface of the bead.

### BRIEF DESCRIPTION OF THE DRAWINGS

The above and other features and advantages of the present invention will become more apparent by describing in detail exemplary embodiments thereof with reference to the attached drawings in which:

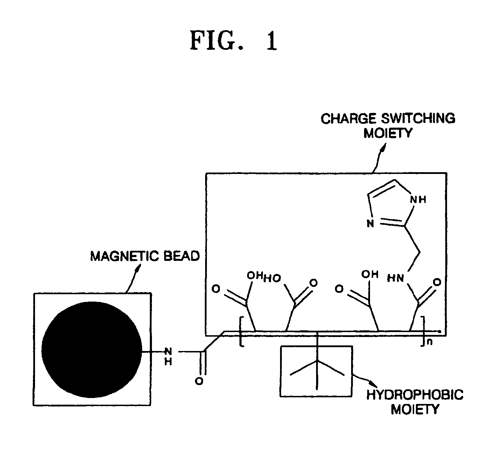

FIG. 1 illustrates a bead used in the method according to an embodiment of the present invention.

### DETAILED DESCRIPTION OF THE INVENTION

According to an embodiment of the present invention, there is provided a method of isolating and purifying nucleic acid from cells or viruses, the method comprising: mixing a sample containing cells or viruses with a solution comprising beads dispersed in a binding buffer to bind the cells or viruses with the beads; separating beads having the cells or viruses bound thereto from the binding buffer and then washing the beads with the buffer; disrupting the cells or viruses bound to the beads to bind nucleic acid released from the cells or viruses to the beads; washing the beads having nucleic acid bound thereto with the binding buffer; and adding a nucleic acid eluting buffer to the beads to elute nucleic acid bound to the beads, wherein a compound represented by Formula 1 below is bound to the surface of the beads: 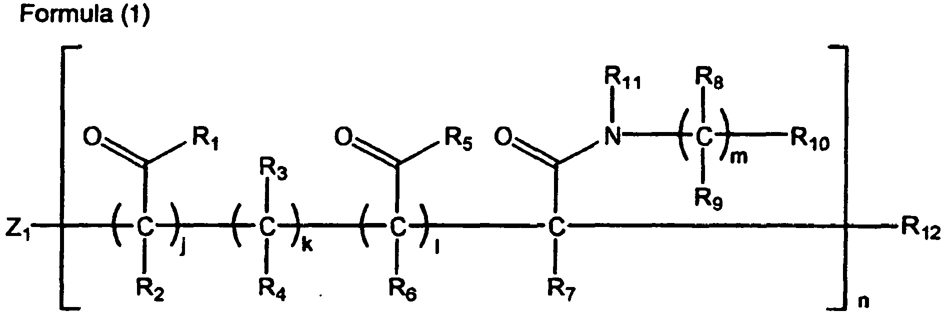 where Z₁ is a carboxyl group or amino group;
R₁, R₂, R₃, R₅, R₆, R₇, R₈, R₉, R₁₁ and R₁₂ are each independently selected from the group consisting of a hydrogen atom, a halogen atom, a hydroxyl group, a substituted or unsubstituted C1-C20 alkyl group, a substituted or unsubstituted C1-C20 alkoxy group, a substituted or unsubstituted C2-C20 alkenyl group, a substituted or unsubstituted C6-C30 aryl group, a substituted or unsubstituted C6-C30 aryloxy group, and a substituted or unsubstituted C3-C20 cycloalkenyl group;
R₄ is a substituted or unsubstituted C4-C20 alkyl group, a substituted or unsubstituted C4-C20 alkoxy group, a substituted or unsubstituted C4-C20 alkenyl group, a substituted or unsubstituted C6-C30 aryl group, a substituted or unsubstituted C6-C30 aryloxy group;
R₁₀ is a nitrogen-containing heteroaryl or heterocyclic group of 3-30 carbon atoms;
j, k, l and m are each independently an integer in the range of 1-10; and
n is an integer in the range of 1-30,000.

In the current embodiment, when a sample containing cells or viruses is mixed with a solution comprising beads dispersed in a binding buffer, the cells or viruses are bound to the hydrophobic part of the surface of a compound bound to the bead. In Formula 1, R₄ is a functional group corresponding to a hydrophobic moiety, and represents a hydrocarbon of at least 4 carbon atoms, preferably, a substituted or unsubstituted C4-C20 alkyl group, a substituted or unsubstituted C4-C20 alkoxy group, a substituted or unsubstituted C4-C20 alkenyl group, a substituted or unsubstituted C6-C30 aryl group, or a substituted or unsubstituted C6-C30 aryloxy group.

If the concentration of the cells or viruses in a sample containing cells or viruses is too low, and thus the volume of the sample containing cells or viruses is large, the cells or viruses have to be bound to a bead and then the bead has to be separated from the sample containing cells or viruses. The bead can be separated using a magnet or an electromagnet, centrifugal separation or the like, but the method of separating the bead is not limited thereto. To disrupt cells or viruses bound to the bead after the bead is separated, the bead is dispersed in a binding buffer. At this time, the volume of the binding buffer may be very small, for example, about 5 µl.

Cells or viruses bound to the dispersed bead are laser-irradiated or heated to disrupt the cells or viruses. The heating for disrupting the cells or viruses can be performed at 70-99°C, preferably at 94-96°C for 1-30 minutes, preferably 3-10 minutes.

Beads that absorb a laser beam, for example, magnetic beads can be used for disrupting cells or viruses by irradiation with a laser beam. A laser beam is irradiated onto a solution containing magnetic beads and the magnetic beads cause an ablation due to the energy of the laser beam. This laser ablation causes shock waves, vapor pressure and heat which is transferred to the virus or cell that is bound to the bead. At this time, physical shocks are also applied to the cell or virus surface. Additionally, the magnetic beads heated by the laser raise the temperature of the solution and directly disrupt the cells. The magnetic beads in the solution do not act as a simple heat conductor but apply thermal, mechanical and physical shocks to the cell or virus surface, thereby efficiently disrupting the cell or virus surface.

The laser beam can be emitted by a pulse laser or continuous wave (CW) laser. At too low laser power, the laser ablation cannot effectively occur. The laser power is 10 mW or more for the CW laser and 1 mJ/pulse or more for the pulse laser. Preferably, the pulse laser is 3 mJ/pulse or more and the CW laser has the power of 100 mW or more. Sufficient energy to disrupt the cells is not transferred when the CW is less than 10 mW and the pulse laser is less than 1 mJ/pulse.

The laser beam should be generated in a specific wavelength range where magnetic beads absorb the energy of the laser. The laser beam has preferably a wavelength in the range of 400 nm or higher, and more preferably in the wavelength range from 750 nm to 1,300 nm. This is because DNA is denaturated or damaged at a wavelength less than 400 nm. The laser beam can also be generated in one or more wavelength ranges. That is, the laser can have one wavelength or two or more different wavelengths within the above range.

The diameter of the magnetic beads is preferably from 50 nm to 1,000 µm, and more preferably from 1 µm to 50 µm. When the diameter of the magnetic beads is less than 50 nm, physical and mechanical shocks are insufficient to cause cell lysis. When the diameter of the magnetic beads is greater than 1,000 µm, it is not suitable for LOC. The magnetic beads can also be a mixture of beads with two or more sizes. That is, the magnetic beads can have equal sizes to each other (i.e. being monodisperse) or be a mixture of beads with different sizes.

When cells or viruses are disrupted by irradiating a laser beam, nucleic acid is released from the cells or viruses, and then the nucleic acid is bound to a bead by a DNA binding moiety of a compound bound to the bead. In Formula 1, R₁₀ is a functional group corresponding to a DNA binding moiety, and is a nitrogen-containing heteroaryl or heterocyclic group of C3-C30, preferably, a pyridinyl group or an imidazolyl group. Preferably, the DNA binding moiety is a group that is positively charged at low pH and uncharged at high pH (e.g. an imidazolyl group).

Subsequently, the bead having nucleic acid bound thereto is washed with a binding buffer. As a result, impurities that are not bound to the bead, for example, cell debris and proteins can be removed.

To elute purified nucleic acid, a nucleic acid eluting buffer is added to the bead, and thus nucleic acid bound to the bead is eluted. Eluting nucleic acid bound to the bead is possible only using a nucleic acid eluting buffer. However, irradiating a laser beam or heating can be simultaneously performed to increase eluting efficiency.

The surface of the bead has a compound represented by Formula 1 bound thereto. In a compound according to an embodiment of the present invention, a carboxyl group or amino group of Z₁, can be bound to the bead by peptide bond.

In Formula 1 above, R₁ and R₅ may each be a hydroxyl group. When R₁ and R₅ is each a hydroxyl group, the resulting carboxylic acid groups represent a nucleic acid eluting moiety of the compound of Formula 1.

The nucleic acid eluting moiety or nucleic acid releasing moiety is necessary for the release or elution of the DNA upon contact of the compound with a nucleic acid eluting buffer. Preferably, the nucleic acid eluting moiety is uncharged (e.g. the carboxylic acid groups are protonated) at low pH (pH 3-5), allowing a nucleic acid to remain bound to the compound, and becomes negatively charged at high pH (pH 8-10), thereby allowing a release of the nucleic acid from the compound. Preferably, the nucleic acid elution or releasing moiety is performing a charge switch at a pH in the range of 7,0 to 10,0.

FIG. 1 illustrates a bead used in the method of the present invention, according to an embodiment of the present invention. In FIG. 1, the bead is a magnetic bead, a DNA binding moiety of a charge switching moiety is an imidazolyl group, and a DNA releasing moiety thereof is a carboxyl group.

In R₁, R₂, R₃, R₅, R₆, R₇, R₈, R₉, R₁₁ and R₁₂, which are substituents used in the compound according to an embodiment of the present invention, each includes an alkyl group that has a straight or branched radical of C1-C20, preferably, a straight or branched radical of C1-C12. Preferably, the alkyl radical is an alkyl radical having 1-6 carbon atoms. Examples of the alkyl radical include methyl, ethyl, n-propyl, isopropyl, n-butyl, isobutyl, sec-butyl, t-butyl, amyl, iso-amyl, hexyl or the like. More preferably, the alkyl radical is an alkyl radical having 1-3 carbon atoms.

In R₁, R₂, R₃, R₅, R₆, R₇, R₈, R₉, R₁₁ and R₁₂, which are substituents used in the compound according to an embodiment of the present invention, each includes alkoxy group that has an oxygen-containing straight or branched radical including an alkyl part of C1-C20. Preferably, the alkoxy radical is an radical having 1-6 carbon atoms. Examples of the alkoxy radical include methoxy, ethoxy, propoxy, butoxy and t-butoxy. More preferably, the alkoxy radical is an alkoxy radical having 1-3 carbon atoms. The alkoxy radical can provide a haloalkoxy radical that is substituted with at least one halo atom such as fluoro, chloro or bromo. More preferably, the haloalkoxy radical is a haloalkoxy radical having 1-3 carbon atoms. Examples of the haloalkoxy radical include fluoromethoxy, chloromethoxy, trifluoromethoxy, trifluoroethoxy, fluoroethoxy and fluoropropoxy.

In R₁, R₂, R₃, R₅, R₆, R₇, R₈, R₉, R₁₁ and R₁₂, which are substituents used in the compound according to an embodiment of the present invention, each includes an alkenyl group that has a carbon-carbon double bond and a C2-C30 straight or branched aliphatic hydrocarbon. The alkenyl group may preferably have 2-12 carbon atoms within a chain, more preferably, 2-6 carbon atoms within a chain. The term "branched" represents that at least one alkyl group or alkenyl group is attached to the straight chain of the alkenyl group. The alkenyl group is unsubstituted, or can be independently substituted by at least one functional group which includes halo, carboxy, hydroxyl, formyl, sulfo, sulfino, carbamoyl, amino and imino, but is not limited thereto. Examples of the alkenyl group include ethenyl, propenyl, carboxyethenyl, carboxypropenyl, sulfinoethenyl and sulfonoethenyl. The alkenyl group may be a substituted or unsubstituted cycloalkenyl group.

The aryl group as used herein, which is used alone or in combination, refers to a carbocyclic aromatic system of 6-20 carbon atoms having one or more rings. The rings may be attached to each other as a pendant group or may be fused. The term "aryl" includes an aromatic radical such as phenyl, naphthyl, tetrahydronaphthyl, indane, and biphenyl. Phenyl is more preferable. The aryl group may have 1-3 substituents such as hydroxy, halo, haloalkyl, nitro, cyano, alkoxy, and alkylamino.

The aryloxy group as used herein refers to aryl-O-. The definition of the "aryl" in the aryloxy group is as described above.

In the method according to an embodiment of the present invention, the binding buffer may have a pH of 3-5. When the pH of the binding buffer is beyond this range, binding efficiency of cells or nucleic acids bound to a bead is decreased. The binding buffer may be phosphate, acetate, citrate, MES or the like, but is not limited thereto. The buffering agent of the binding buffer may have a concentration of 10-1,000 mM. When this concentration in the binding buffer is less than this range, efficiency of binding of cells or nucleic acids to a bead and efficiency of washing impurities such as cell debris and proteins are reduced. When the concentration of the binding buffer is greater than this range, preparing the binding buffer is difficult.

In the method according to an embodiment of the present invention, the nucleic acid eluting buffer may have a pH of 8-10. When the pH of the nucleic acid eluting buffer is less than this range, eluting efficiency of nucleic acid is reduced. When the pH of the nucleic acid eluting buffer is greater than this range, subsequent processes can be affected. The nucleic acid eluting buffer can be phosphate, HEPES, Tris or the like, but is not limited thereto. The buffering agent in the nucleic acid eluting buffer may have a concentration of 10-1,000 mM. When this concentration in the nucleic acid eluting buffer is beyond this range, eluting efficiency of nucleic acid bound to a bead is reduced, and subsequent processes can be also affected.

In the method according to an embodiment of the present invention, the bead can be a magnetic bead, a silica bead, a polystyrene bead, a glass bead, a cellulose bead or the like, but is not limited thereto. Preferably, the bead is a magnetic bead when a laser is used for disrupting cells or viruses bound to a bead, since the magnetic bead readily absorbs the light of the laser. This absorption can produce heat or lead to laser ablation. On the other hand, in the case of a silica bead, a polystyrene bead, a glass bead, or a cellulose bead that may not absorb laser light, disruption of cells or viruses may be performed by heating.

In an embodiment of the present invention, the sample containing cells or viruses can be saliva, urine, blood, serum, cell culture or the like, but is not limited thereto. The sample can be any solution having nucleic acids, such as a solution containing animal cells, plant cells, bacteria, viruses, phage and the like.

According to another embodiment of the present invention, there is provided an apparatus comprising a bead according to the present invention for continuously performing isolation and purification of nucleic acid, the apparatus comprising: a cell or virus disruption micro chamber having a sample inlet through which a sample containing cells or viruses is introduced; a bead dispersion storing part being in a fluid communication with the cell or virus disruption micro chamber through a micro channel which supplies the bead dispersion to the micro chamber through the micro channel; a binding buffer storing part being in a fluid communication with the cell or virus disruption micro chamber through a micro channel which supplies the binding buffer to the micro chamber through the micro channel; a nucleic acid eluting buffer storing part being in a fluid communication with the cell or virus disruption micro chamber through a micro channel which supplies the nucleic acid eluting buffer to the micro chamber through the micro channel; and a laser generation part attached to the cell or virus disruption micro chamber and supplying a laser beam to the micro chamber.

The micro chamber that disrupts cells or viruses includes an inlet that introduces a sample including cells or viruses, and the sample including cells or viruses is supplied through the inlet. The samples are thoroughly mixed with magnetic beads. The thorough mixing of the samples and the magnetic beads is achieved by a vibrator. A laser irradiates the mixture while the mixture is vibrated. A cell disruption chamber window should be composed of a material through which the laser can sufficiently pass. The magnetic beads exposed to the laser transform light to heat, i.e. laser ablation occurs. Heat, vibration, shock wave, vapor pressure, etc. are efficiently transferred due to effective heat transfer and collision of the magnetic beads with cells by continuous vibration.

The vibrator can include sonicators, vibrators using a magnetic field, vibrators using an electric field, mechanical vibrators such as a vortex etc., or piezoelectric materials. The vibrator is attached to the micro chamber and can be any device capable of vibrating the mixed solution of the cells and the magnetic beads.

In the apparatus according to an embodiment of the present invention, the bead is a magnetic bead, and the apparatus can further include an electromagnet that is attached to the micro chamber and fixes the magnetic beads. The electromagnet is attached to the micro chamber to immobilize magnetic beads in a predetermined space of the micro chamber, and then a sample containing cells or viruses is flowed over the immobilized magnetic beads to contact the magnetic beads, thereby binding the cells or viruses with the magnetic beads. In the case of not using the electromagnet, additional steps of mixing magnetic beads with a sample containing cells or viruses to bind the cells or viruses with the magnetic beads, and then separating the magnetic beads to which cells or viruses are bound from the sample and dispersing the magnetic beads in a binding buffer are required. However, when the electromagnet is used, these additional steps are unnecessary, and thus the number of steps for separating cells or viruses can be reduced. For the purpose of the lab-on-chip (LOC) implementation, the magnetic beads are removed by the electromagnet after disruption of cells or viruses and nucleic acid purification are completed so that the purified nucleic acid solution can directly run to a PCR chamber without performing separation of the magnetic beads. The beads should be magnetized in order to be removed by the electromagnet.

The apparatus according to an embodiment of the present invention can further include a DNA amplification chamber being in a fluid communication with the micro chamber through a microchannel. For the purpose of the LOC implementation, an amplification system of the purified DNA is necessary. The purified DNA can be detected using a spectrophotometer, an electrochemical method, electrochomiluminescence, radiation and fluorescent label, a real-time PCR method, and the like. The PCR method is most suitable to sufficiently amplify a desired DNA. Other DNA amplification methods can be applied and direct detection through the real-time PCR method, etc. is also possible.

The laser generating part can generate a pulse laser or continuous wave (CW) laser. At too low laser power, the laser ablation cannot effectively occur.

The laser power is 10 mW or more for the CW laser and 1 mJ/pulse or more for the pulse laser. Preferably, the pulse laser is 3 mJ/pulse or more and the CW laser has the power of 100 mW or more. Sufficient energy for disrupting the cells is not transferred, when the CW is less than 10 mW and the pulse laser is less than 1 mJ/pulse.

The laser should be generated in a specific wavelength range where magnetic beads absorb the energy of the laser. The laser is generated preferably in the wavelength range of 400 nm or more, and more preferably in the wavelength range from 750 nm to 1,300 nm. This is because DNA is denatured or damaged at a wavelength less than 400 nm. The laser can also be generated in one or more wavelength ranges. That is, the laser can have one wavelength or two or more different wavelengths within the above range.

According to another embodiment of the present invention, there is provided a lab-on-a-chip comprising the apparatus according to the present invention for continuously performing nucleic acid isolation and purification. Each functional element of the apparatus for isolating and purifying nucleic acid can be designed for a process-on-a-chip or a lab-on-a-chip, using known microfluldic techniques and microelectromechanical system (MEMS) devices.

According to another embodiment of the present invention, there is provided a bead for isolating and purifying nucleic acid from cells or viruses, wherein a compound represented by Formula 1 below is bound to the surface of the bead. 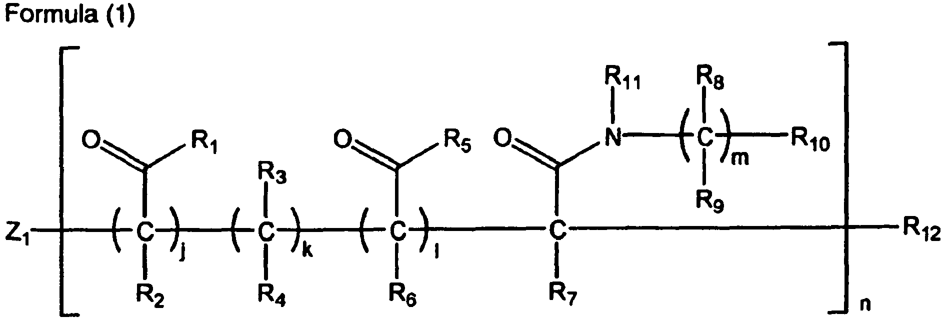 where each of Z₁, R₁, R₂, R₃, R₄, R₅, R₆, R₇, R₈, R₉, R₁₀, R₁₁, R₁₂, j, k, l, m and n is the same as defined above.

In the bead according to the current embodiment of the present invention, R₁₀ is a pyridinyl group or imidazolyl group, and each of R₁ and R₅ is a hydroxyl group.

The present invention will now be described in greater detail with reference to the following examples. The following examples are for illustrative purposes only and are not intended to limit the scope of the invention.

### Examples

### Example 1: Bead surface treatment

### 1) Coupling of polyanhydrides (a functional group was introduced for introducing a hydrophobic functional group and an ionizable functional group)

The magnetic beads of 1 ml of a solution containing magnetic beads having an amine functional group, Dynabeads® M-270 Amine (Invitrogen), were washed with 1 ml of N-methyl-2-pyrolidone (NMP) three times. The magnetic beads were separated from the solution using a magnet, and then 1 ml of 200 mM (this molarity being based on the monomeric repeating unit of the polymer) polyanhydride (poly(isobutyl-alt-maleic anhydride)) having an average molecular weight of 50,000, which was dissolved in NMP, was added to the magnetic bead. Thereafter, the resulting product was mixed for one hour and washed with NMP three times.

### 2) Introduction of a positive ionizable group

A solvent was removed from the resulting magnetic bead solution of the process 1). Then, 1 ml of a solution of 3 mM 1-(3-aminopropyl)imidazol and 6 mM triethylamine dissolved in NMP was added to the magnetic bead. The resulting product was mixed for one hour, and then washed with NMP three times.

### 3) Introduction of a negative ionizable group

A solvent was removed from the resulting magnetic bead solution of the process 2). 1 ml of a 0.01 N NaOH solution was added to the magnetic bead. Then, the resulting product was mixed for 30 minutes and washed with 1 ml of distilled water five times. Lastly, the resulting product was dispersed in 1 ml of sterilized triple distilled water and stored in the cold.

### Example 2: Cell concentration using the method according to an embodiment of the present invention

50 µl of the solution comprising beads dispersed in distilled water (prepared in Example 1) was washed two times with 100 µl of a cell binding buffer (100 mM sodium acetate, pH 4.0), and then dispersed in 100 µl of the same buffer. 10 µl of E. coli BL21(1.0 OD₆₀₀) in 1X PBS was mixed with 40 µl of a 1X PBS buffer, or 40 µl of urine, or 40 µl of blood, respectively. Then, the resulting products were mixed with a prepared bead solution for two minutes. Subsequently, the beads were separated from the solution using a magnet. Each of a cell solution of before mixing with the bead solution (control) and a cell solution of after mixing with the bead solution and removal of the beads was diluted to 1/10,000 with a 1X PBS solution. Then, the diluted cell solutions were plated on 3M™ Petrifilm™ (3M) and the number of the grown colonies for the cell solutions was counted to calculate binding efficiency.

**Table 1**

| Sample | PBS buffer | Urine | Blood |
|---|---|---|---|
| Binding Efficiency | >99% | >99% | 25.0±5.7% |

As can be seen in Table 1, in the samples with PBS buffer or urine, E. coli cells are efficiently bound to the beads. However, efficiency of binding of the E. coli cells to the bead in the sample with blood is relatively low. It is considered that this is because a relatively large amount of material that inhibits E. coli cells from being bound to the bead exists in the blood (e.g. the blood cells themselves).

### Example 3: Cell disruption and nucleic acid purification using the method according to an embodiment of the present invention

The beads concentrated with E. coli cells of Example 2 were dispersed in 5 µl of binding buffer, and the resulting solution was placed in a microchip for PCR in a TMC-1000 (Samsung Techwin). Then, a laser (Hamamatsu 8446-72, 1W, 808nm) irradiated the solution for 40 seconds, and the supernatant was separated from the beads to quantify DNA of the supernatant using PicoGreen® (Invitrogen). The beads were dispersed again in 5µl of binding buffer, and mixed for one minute. Then, the resulting supernatant was removed from the beads and the beads were dispersed in 5µl of elution buffer (100 mM Tris, pH 9.5), and then the resulting bead dispersion was placed in a microchip for PCR in a TMC-1000 (Samsung Techwin). 1.5 W of laser was irradiated to the solution for 40 seconds, and then the supernatant was separated from the beads to quantify DNA in the supernatant (eluate) using PicoGreen®. The results of DNA quantification of the supernatant before DNA elution (after cell disruption) and the eluate after DNA elution are shown in Table 2 below.

**Table 2**

| Sample | PBS buffer | Urine |
|---|---|---|
| After cell disruption | <0.1 ng/µℓ | <0.1 ng/µℓ |
| After DNA elution | 6.1±1.1 ng/µℓ | 5.0±0.9 ng/µℓ |

As can be seen in Table 2, virtually no DNA is detectable in the supernatant unless the E. coli cells bound to the magnetic bead are disrupted and nucleic acids are eluted from the E. coli cells. Thus, all of the DNA is bound to the beads after cell disruption. In addition, it can be seen that a large amount of DNA is found in the eluate after DNA elution.

Therefore, it is confirmed that after cells bound to a bead are disrupted, nucleic acid can be eluted only by exchange of a buffer without a change of the surface of the bead.

### Example 4: Purity measurement of the purified nucleic acid using the method according to an embodiment of the present invention

Values of A₂₆₀/A₂₈₀ for the finally eluted solution of Example 3 were measured using NanoDrop® (NanoDrop Technologies). An experiment was performed for a control in which only cell disruption was performed without nucleic acid purification such that 5 µl of a 1X PBS buffer containing E. coli (OD₆₀₀=2.0) was added to the beads from 100 µl of Dynabead® MyOne™ carboxylic acid (Invitrogen) and a laser beam was irradiated to the resulting mixture for 40 seconds and then the supernatant was isolated. Values of A₂₆₀/A₂₈₀ with respect to samples of the present invention in which DNA purification was performed after cell disruption and samples of a control in which DNA purification was not performed are shown in Table 3 below.

**Table 3**

| Sample | Present Invention | Control |
|---|---|---|
| A₂₆₀/A₂₈₀ | 1.92±0.10 | 2.74±0.34 |

As can be seen in Table 3, a sample of the present invention, in which a nucleic acid purification process was performed, has a value of A₂₆₀/A₂₈₀ in the range of 1.8-2.0. Therefore, it can be seen that the sample of the present invention is purified to a higher extent compared to the control in which the nucleic acid purification process was not performed.

### Example 5: Real-time PCR performed using nucleic acid purified by the method according to an embodiment of the present invention

To additionally confirm the effect of DNA purification, a real-time PCR was performed using a DNA solution prepared in Example 3. A PCR master mixture was prepared containing 2X solgent PCR buffer, 0.6 U/µl of Taq polymerase (Solgent Co. Ltd.), 0.4 mM of dNTP, 0.4 µM of a forward primer, 0.4 µM of a reverse primer, 10 mM of MgCl₂, and 2X SYBR-green. Then, the PCR master mixture was mixed with the DNA solution prepared in Example 3 in a ratio of 1:1, and with the resulting mixture, PCR was performed using TMC-1000. Conditions of PCR were as follows: initial denaturation at 94°C for one minute, and then 40 cycles of 94°C for 5 seconds, 62°C for 5 seconds, and 72°C for 40 seconds. A region to be amplified is a 16s rRNA gene. The primers used had the sequences as follows.
Forward primer: 5'-YCC AKA CTC CTA CGG GAG GC-3'(SEQ ID NO: 1)
Reverse primer: 5'-GTA TTA CCG CTT CTG CTG GCA C-3'(SEQ ID NO: 2)

Table 4 below shows the results of real-time PCR performed using DNA of before and after purification, represented as Ct. Ct represents the number of cycles at which detectable fluorescence signals are detected in a real-time PCR. That is, the higher an initial concentration of DNA is, the more fluorescence signals can be detected at a low Ct value. Ct is also correlates with DNA purification. That is, the higher the DNA purity is, the lower the Ct value is, and the lower the DNA purity is, the higher the Ct value is. Therefore, it can be seen that the lower the Ct values are, the more purified is the DNA in a solution.

**Table 4**

| Sample | Urine | PBS buffer |
|---|---|---|
| Present Invention(purified) | 19.86±0.59 | 23.92±0.32 |
| Control(not purified) | 27.08±1.54 | 33.86±0.88 |

As can be seen in Table 4, samples of the present invention in which DNA purification was performed have much lower Ct values than those of control samples, regardless of sample type, due to a DNA purification effect of the present invention.

Therefore, it is confirmed that DNA subjected to the purification of the present invention is purified to a higher extent than DNA in a control which was not subjected to the purification of the present invention.

After PCR was completed, a solution was separated from a chip to analyze a PCR product using LabChip® (Aglient). Table 5 represents results of real-time PCR with DNA of before and after purification as a PCR product concentration.

**Table 5**

| Sample | Urine | PBS buffer |
|---|---|---|
| Present Invention(purified) | 13.9±1.7 ng/µℓ | 11.9±3.7 ng/µℓ |
| Control(not purified) | 7.8±1.7 ng/µℓ | 1.9±0.5 ng/µℓ |

As can be seen in Table 5, samples of the present invention in which DNA purification is performed produce much more amplicon compared to samples of a control in which the DNA was not purified, regardless of sample types, due to an effect of DNA purification of the present invention.

According to the method of the present invention, cell concentration, cell disruption and DNA purification can be performed without changing surfaces using a single surface that can bind cells and DNA under the same condition and release DNA under high pH conditions. Since the whole process is performed using a single surface, a system can be easily manufactured, and can be also widely used in CDs, microchips and the like.

While the present invention has been particularly shown and described with reference to exemplary embodiments thereof, it will be understood by those of ordinary skill in the art that various changes in form and details may be made therein without departing from the scope of the present invention as defined by the following claims.
## Claims

1. A method of isolating and purifying nucleic acid from cells or viruses, the method comprising:
mixing a sample containing cells or viruses with a solution comprising beads dispersed in a binding buffer to bind the cells or viruses with the beads;
separating beads having the cells or viruses bound thereto from the binding buffer and then washing the bead with the buffer;
disrupting the cells or viruses bound to the beads to bind nucleic acid released from the cells or viruses with the beads;
washing the beads having nucleic acid bound thereto with the buffer; and
adding a nucleic acid eluting buffer to the beads to elute nucleic acid bound to the beads,
wherein a compound represented by Formula 1 below is bound to the surface of the beads: 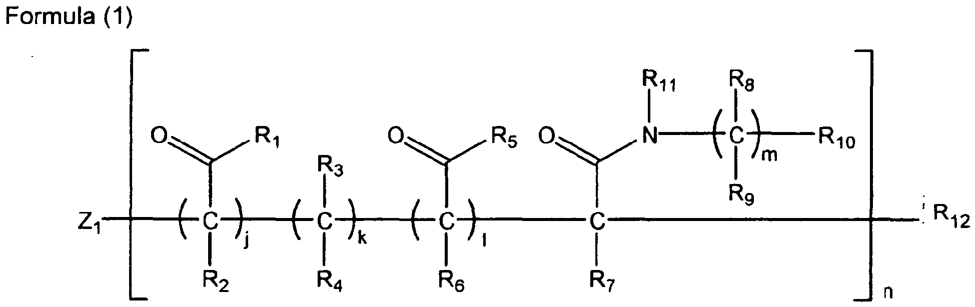 where Z₁ is a carboxyl group or amino group;
R₁, R₂, R₃, R₅, R₆, R₇, R₈, R₉, R₁₁ and R₁₂ are each independently selected from the group consisting of a hydrogen atom, a halogen atom, a hydroxyl group, a substituted or unsubstituted C1-C20 alkyl group, a substituted or unsubstituted C1-C20 alkoxy group, a substituted or unsubstituted C2-C20 alkenyl group, a substituted or unsubstituted C6-C30 aryl group, and a substituted or unsubstituted C6-C30 aryloxy group, and a substituted or unsubstituted C3-C20 cycloalkenyl group;
R₄ is a substituted or unsubstituted C4-C20 alkyl group, a substituted or unsubstituted C4-C20 alkoxy group, a substituted or unsubstituted C4-C20 alkenyl group, a substituted or unsubstituted C6-C30 aryl group, or a substituted or unsubstituted C6-C30 aryloxy group;
R₁₀ is a nitrogen-containing heteroaryl or heterocyclic group of 3-30 carbon atoms;
j, k, I and m are each independently an integer in the range of 1-10; and
n is an integer in the range of 1-30,000.

2. The method of claim 1, wherein the disrupting of the cells or viruses is performed by heating or by irradiation with a laser beam.

3. The method of claim 2, wherein the laser beam is emitted by a pulse laser or continuous wave (CW) laser.

4. The method of claim 3, wherein the pulse laser has a power of 3 mJ/pulse or more, and the CW laser has a power of 100 mW or more.

5. The method of claim 3 or 4, wherein the laser beam is generated in a wavelength range of 400 nm or higher.

6. The method of claims 1 to 5, wherein the binding buffer has a pH of 3-5.

7. The method of claims 1 to 6, wherein the nucleic acid eluting buffer has a pH of 8-10.

8. The method of claims 1 to 7, wherein laser irradiation or heating is simultaneously performed with the adding of nucleic acid eluting buffer.

9. The method of claims 1 to 8, wherein the bead is a magnetic bead, a silica bead, a polystyrene bead, a glass bead, or a cellulose bead.

10. The method of claims 1 to 9, wherein R₁₀ is a pyridinyl group or an imidazolyl group.

11. The method of claims 1 to 10, wherein R₁ and R₅ are each a hydroxyl group.

12. The method of claims 1 to 11, wherein the sample containing cells or viruses is saliva, urine, blood, serum, or cell culture.

13. An apparatus comprising a bead according to any one of claims 20 to 22 for continuously performing isolation and purification of nucleic acid, the apparatus comprising:
a cell or virus disruption micro chamber having a sample inlet through which a sample containing cells or viruses is introduced;
a bead dispersion storing part being in a fluid communication with the cell or virus disruption micro chamber through a micro channel and supplying a bead dispersion to the micro chamber through the micro channel;
a binding buffer storing part being in a fluid communication with the cell or virus disruption micro chamber through a micro channel and supplying the binding buffer to the micro chamber through the micro channel;
a nucleic acid eluting buffer storing part being in a fluid communication with the cell or virus disruption micro chamber through a micro channel and supplying the nucleic acid eluting buffer to the micro chamber through the micro channel; and
a laser generation part attached to the cell or virus disruption micro chamber and supplying a laser beam to the micro chamber.

14. The apparatus of claim 13, wherein the bead is a magnetic bead, and further comprises an electromagnet that is attached to the micro chamber, and immobilizes the magnetic bead.

15. The apparatus of claim 13 or 14, further comprising a DNA amplification chamber being in a fluid communication with the micro chamber through a micro channel.

16. The apparatus of claim 13 or 15, wherein a laser produced by the laser generation part comprises a pulse laser or continuous wave (CW) laser.

17. The apparatus of claim 16, wherein the pulse laser has a power of 3 mJ/pulse or more, and the CW laser has a power of 100 mW or more.

18. The apparatus of claim 16 or 17, wherein the laser beam is generated in a wavelength range of 400 nm or more.

19. A lab-on-a-chip comprising the apparatus according to claims 13 to 18.

20. A bead for isolating and purifying nucleic acid from a cell or virus, wherein a material represented by Formula 1 below is bound to the surface of the bead: 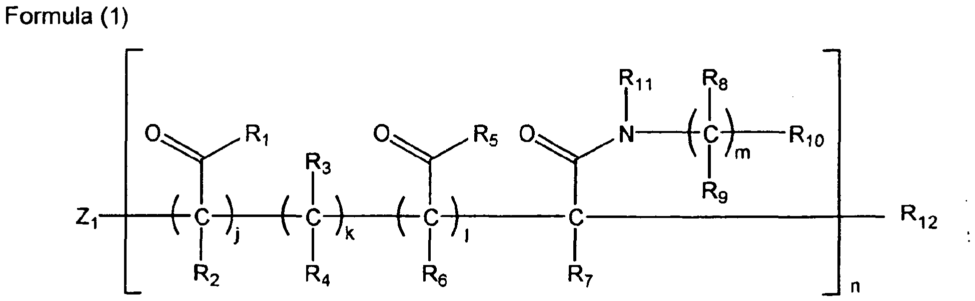 where Z₁, R₁, R₂, R₃, R₄, R₅, R₆, R₇, R₈, R₉, R₁₀, R₁₁, R₁₂, j, k, I, m and n are the same as defined in claim 1.

21. The bead of claim 20, wherein R₁₀ is a pyridinyl group or an imidazolyl group.

22. The bead of claim 20 or 21, wherein R₁ and R₅ are each a hydroxyl group.

## Patentansprüche

1. Verfahren zur Isolation und Reinigung von Nukleinsäure aus Zellen oder Viren, wobei das Verfahren umfasst:
Mischen einer Probe, welche Zellen oder Viren enthält, mit einer Lösung umfassend Kugeln aufgelöst in einem Bindepuffer, um die Zellen oder Viren an die Kugeln zu binden;
Trennen der Kugeln, an welchen die Zellen oder Viren gebunden sind, aus dem Bindepuffer und anschließend Waschen der Kugeln mit dem Puffer;
Zerstören bzw. Zerkleinern der Zellen oder Viren, welche an die Kugeln gebunden sind, um von den Zellen oder Viren freigegebene Nukleinsäure an die Kugeln zu binden,
Waschen der Kugeln, an welche die Nukleinsäure gebunden ist, mit dem Puffer; und
Zugeben eines Nukleinsäure eluierenden Puffers zu den Kugeln, um die an die Kugeln gebundene Nukleinsäure zu eluieren,
wobei eine Verbindung, dargestellt durch die nachfolgende Formel 1, an die Oberfläche der Kugeln gebunden ist: 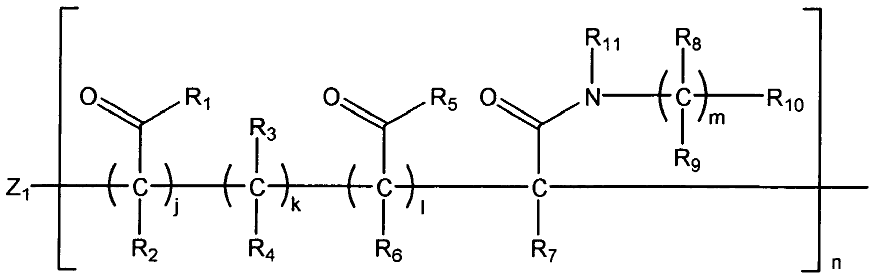 wobei Z₁ eine Carboxylgruppe oder Aminogruppe ist;
R₁, R₂, R₃, R₅, R₆, R₇, R₈, R₉, R₁₁ and R₁₂ jeweils unabhängig gewählt sind aus der Gruppe bestehend aus einem Wasserstoffatom, einem Halogenatom, einer Hydroxylgruppe, einer substituierten oder unsubstituierten C1-C20 Alkylgruppe, einer substituierten oder unsubstituierten C1-C20 Alkoxygruppe, einer substituierten oder unsubstituierten C2-C20
Alkylengruppe, einer substituierten oder unsubstituierten C6-C30 Arylgruppe, und einer substituierten oder unsubstituierten C6-C30 Aryloxygruppe und einer substituierten oder unsubstituierten C3-C20 Cycloalkenylgruppe;
R₄ eine substituierte oder unsubstituierte C4-C20 Alkylgruppe, eine substituierte oder unsubstituierte C4-C20 Alkoxygruppe, eine substituierte oder unsubstituierte C4-C20 Alkenylgruppe, eine substituierte oder unsubstituierte C6-C30 Arylgruppe, oder eine substituierte oder unsubstituierte C6-C30 Aryloxygruppe ist;
R₁₀ eine stickstoffhaltige Heteroarylgruppe oder heterozyklische Gruppe mit 3-30 Kohlenstoffatomen ist;
j, k, l und m jeweils unabhängig eine ganze Zahl in dem Bereich von 1 bis 10 sind und
n eine ganze Zahl in dem Bereich von 1 - 30.000 ist.

2. Verfahren nach Anspruch 1, wobei das Zerstören der Zellen oder Viren durch Erwärmen oder durch Bestrahlen mit einem Laserstrahl durchgeführt wird.

3. Verfahren nach Anspruch 2, wobei der Laserstrahl von einem Pulslaser oder einem kontinuierlichen Wellen- (CW) Laser emittiert wird.

4. Verfahren nach Anspruch 3, wobei der Pulslaser eine Leistung von 3 mJ/Puls oder mehr aufweist und der CW-Laser eine Leistung von 100 mW oder mehr aufweist.

5. Verfahren nach Anspruch 3 oder 4, wobei der Laserstrahl in einem Wellenlängenbereich von 400 nm oder mehr erzeugt wird.

6. Verfahren nach Anspruch 1 bis 5, wobei der Bindepuffer einen pH-Wert von 3 - 5 aufweist.

7. Verfahren nach Anspruch 1 bis 6, wobei der die Nukleinsäure eluierende Puffer einen pH-Wert von 8 bis 10 aufweist.

8. Verfahren nach Anspruch 1 bis 7, wobei die Laserbestrahlung oder die Erwärmung gleichzeitig mit der Zugabe des Nukleinsäure eluierenden Puffers durchgeführt wird.

9. Verfahren nach Anspruch 1 bis 8, wobei die Kugel eine Magnetkugel, eine Siliziumdioxidkugel, eine Polystyrolkugel, eine Glaskugel oder eine Cellulosekugel ist.

10. Verfahren nach Anspruch 1 bis 9, wobei R₁₀ eine Pyridinylgruppe oder eine Imidazolylgruppe ist.

11. Verfahren nach Anspruch 1 bis 10, wobei R₁ und R₅ jeweils eine Hydroxylgruppe sind.

12. Verfahren nach Anspruch 1 bis 11, wobei die Probe enthaltenden Zellen oder Viren Speichel, Urin, Blut, Serum oder Zellkultur ist.

13. Vorrichtung umfassend eine Kugel nach einem der Ansprüche 20 bis 22 zur kontinuierlichen Durchführung von Isolierung und Reinigung von Nukleinsäure, wobei die Vorrichtung umfasst:
eine Zell- oder Viruszerstörungsmikrokammer mit einem Probeneinlass, durch welchen eine Probe enthaltend Zellen oder Viren eingeführt ist;
einen Kugeldispersionsspeicherteil, welcher sich durch einen Mikrokanal in einer Fluidverbindung mit der Zell- oder Viruszerstörungsmikrokammer befindet und eine Kugeldispersion durch den Mikrokanal in die Mikrokammer führt;
einen Bindepufferspeicherteil, welcher sich durch einen Mikrokanal in einer Fluidverbindung mit der Zell- oder Viruszerstörungsmikrokammer befindet und den Bindepuffer durch den Mikrokanal in die Mikrokammer einführt;
einen Nukleinsäure eluierenden Pufferspeicherteil, welcher sich durch einen Mikrokanal in einer Fluidverbindung mit der Zell- oder Viruszerstörungsmikrokammer befindet und den Nukleinsäure eluierenden Puffer durch den Mikrokanal in die Mikrokammer führt; und
einen Lasererzeugungsteil, befestigt an der Zell- oder Viruszerstörungsmikrokammer, welcher einen Laserstrahl in die Mikrokammer führt.

14. Vorrichtung nach Anspruch 13, wobei die Kugel eine Magnetkugel ist und des Weiteren einen Elektromagnet umfasst, welcher an der Mikrokammer befestigt ist und die Magnetkugel immobilisiert.

15. Vorrichtung nach Anspruch 13 oder 14, des Weiteren umfassend eine DNA-Verstärkungskammer, welche sich durch einen Mikrokanal in einer Fluidverbindung mit der Mikrokammer befindet.

16. Vorrichtung nach Anspruch 13 oder 15, wobei ein von dem Lasererzeugungsteil erzeugter Laser einen Pulslaser oder einen kontinuierlichen Wellen- (CW) Laser umfasst.

17. Vorrichtung nach Anspruch 16, wobei der Pulslaser eine Leistung von 3 mJ je Puls oder mehr aufweist und der CW-Laser eine Leistung von 100 mW oder mehr aufweist.

18. Vorrichtung nach Anspruch 16 oder 17, wobei der Laserstrahl in einem Wellenlängenbereich von 400 nm oder mehr erzeugt ist.

19. Lab-on-Chip-Anwendung umfassend die Vorrichtung nach Anspruch 13 bis 18.

20. Kugel zum Isolieren und Reinigen von Nukleinsäure aus einer Zelle oder einem Virus, wobei ein Material dargestellt durch die nachfolgende Formel 1 an die Oberfläche der Kugel gebunden ist: 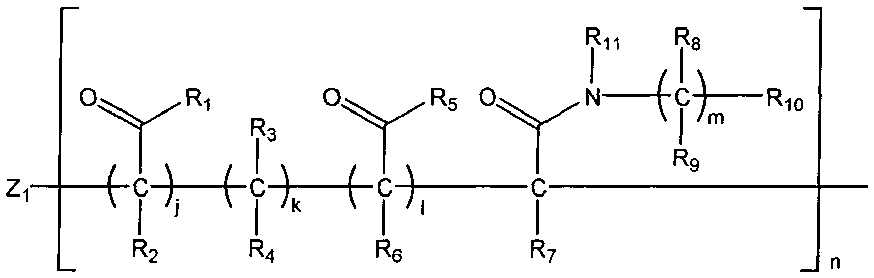 wobei Z₁, R₁. R₂, R₃, R₄, R₅, R₆, R₇, R₈, R₉, R₁₀, R₁₁, R₁₂, j, k, l, m und n wie in Anspruch 1 definiert sind.

21. Kugel nach Anspruch 20, wobei R₁₀ eine Pyridinylgruppe oder eine Imidazolylgruppe ist.

22. Kugel nach Anspruch 20 oder 21, wobei R₁ und R₅ jeweils eine Hydroxylgruppe sind.

## Revendications

1. Procédé pour isoler et purifier de l'acide nucléique à partir de cellules ou de virus, ledit procédé comprenant :
le mélange d'un échantillon contenant des cellules ou des virus avec une solution comprenant des billes dispersées dans un tampon de liaison pour lier les cellules ou les virus aux billes ;
la séparation des billes auxquelles se sont liés des cellules ou des virus, du tampon de liaison, puis le lavage des billes avec le tampon ;
la dissociation des cellules ou des virus liés aux billes pour lier l'acide nucléique libéré par les cellules ou les virus aux billes ;
le lavage des billes auxquelles s'est lié de l'acide nucléique avec le tampon ; et
l'ajout d'un tampon d'élution d'acide nucléique aux billes pour éluer l'acide nucléique lié aux billes,
dans lequel un composé représenté par la Formule 1 ci-dessous est lié à la surface des billes : 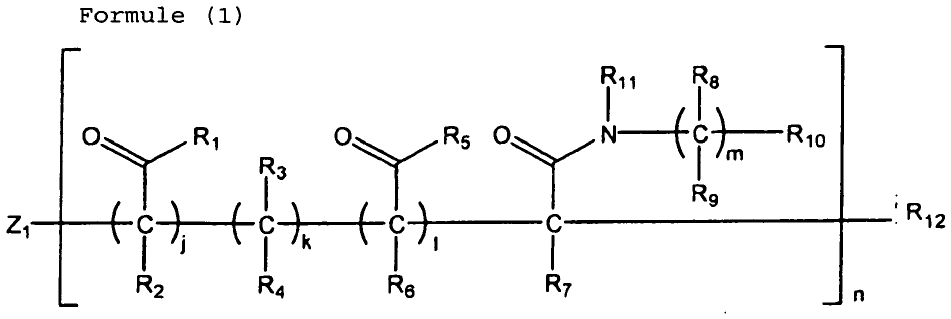 où Z₁ est un groupe carboxyle ou groupe amino ;
R₁, R₂, R₃, R₅, R₆, R₇, R₈, R₉, R₁₁ et R₁₂ sont chacun indépendamment choisis dans le groupe constitué par un atome d'hydrogène, un atome d'halogène, un groupe hydroxyle, un groupe alkyle en C₁-C₂₀ substitué ou non substitué, un groupe alcoxy en C₁-C₂₀ substitué ou non substitué, un groupe alcényle en C₂-C₂₀ substitué ou non substitué, un groupe aryle en C₆-C₃₀ substitué ou non substitué, et un groupe aryloxy en C₆-C₃₀ substitué ou non substitué, et un groupe cycloalcényle en C₃-C₂₀ substitué ou non substitué ;
R₄ est un groupe alkyle en C₄-C₂₀ substitué ou non substitué, un groupe alcoxy en C₄-C₂₀ substitué ou non substitué, un groupe alcényle en C₄-C₂₀ substitué ou non substitué, un groupe aryle en C₆-C₃₀ substitué ou non substitué, ou un groupe aryloxy en C₆-C₃₀ substitué ou non substitué ;
R₁₀ est un groupe hétéroaryle ou hétérocyclique azoté ayant 3 à 30 atomes de carbone ;
j, k, l et m sont chacun indépendamment un entier compris entre 1 et 10 ; et
n est un entier compris entre 1 et 30 000.

2. Procédé selon la revendication 1, dans lequel la dissociation des cellules ou des virus est réalisée par chauffage ou par irradiation avec un faisceau laser.

3. Procédé selon la revendication 2, dans lequel le faisceau laser est émis par un laser à impulsions ou par un laser à onde continue (CW).

4. Procédé selon la revendication 3, dans lequel le laser à impulsions a une énergie égale ou supérieure à 3 mJ/impulsion, et le laser CW a une puissance égale ou supérieure à 100 mW.

5. Procédé selon la revendication 3 ou 4, dans lequel le faisceau laser est généré dans une gamme de longueurs d'onde de 400 nm ou plus.

6. Procédé selon les revendications 1 à 5, dans lequel le tampon de liaison a un pH de 3-5.

7. Procédé selon les revendications 1 à 6, dans lequel le tampon d'élution d'acide nucléique a un pH de 8-10.

8. Procédé selon les revendications 1 à 7, dans lequel l'irradiation par laser ou le chauffage est réalisé conjointement avec l'ajout de tampon d'élution d'acide nucléique.

9. Procédé selon les revendications 1 à 8, dans lequel la bille est une bille magnétique, une bille de silice, une bille de polystyrène, une bille de verre, ou une bille de cellulose.

10. Procédé selon les revendications 1 à 9, dans lequel R₁₀ est un groupe pyridinyle ou un groupe imidazolyle.

11. Procédé selon les revendications 1 à 10, dans lequel R₁ et R₅ sont chacun un groupe hydroxyle.

12. Procédé selon les revendications 1 à 11, dans lequel l'échantillon contenant des cellules ou des virus est de la salive, de l'urine, du sang, du sérum, ou une culture cellulaire.

13. Appareil comprenant une bille selon l'une quelconque des revendications 20 à 22 pour réaliser en continu l'isolement et la purification d'acide nucléique, ledit appareil comprenant :
une microchambre de dissociation de cellules ou de virus, possédant une entrée d'échantillon par laquelle un échantillon contenant des cellules ou des virus est introduit ;
une partie de stockage d'une dispersion de billes, ladite partie étant en communication fluidique avec la microchambre de dissociation de cellules ou de virus par l'intermédiaire d'un microcanal et approvisionnant la microchambre en dispersion de billes par l'intermédiaire du microcanal ;
une partie de stockage d'un tampon de liaison, ladite partie étant en communication fluidique avec la microchambre de dissociation de cellules ou de virus par l'intermédiaire d'un microcanal et approvisionnant la microchambre en tampon de liaison par l'intermédiaire du microcanal ;
une partie de stockage d'un tampon d'élution d'acide nucléique, ladite partie étant en communication fluidique avec la microchambre de dissociation de cellules ou de virus par l'intermédiaire d'un microcanal et approvisionnant la microchambre en tampon d'élution d'acide nucléique par l'intermédiaire du microcanal ; et
une partie de génération de laser fixée à la microchambre de dissociation de cellules ou de virus et approvisionnant la microchambre en faisceau laser.

14. Appareil selon la revendication 13, dans lequel la bille est une bille magnétique, et qui comprend en outre un électroaimant qui est fixé à la microchambre, et immobilise la bille magnétique.

15. Appareil selon la revendication 13 ou 14, comprenant en outre une chambre d'amplification d'ADN qui est en communication fluidique avec la microchambre par l'intermédiaire d'un microcanal.

16. Appareil selon la revendication 13 ou 15, dans lequel un laser produit par la partie de génération de laser comprend un laser à impulsions ou un laser à onde continue (CW).

17. Appareil selon la revendication 16, dans lequel le laser à impulsions a une énergie égale ou supérieure à 3 mJ/impulsion, et le laser CW a une puissance égale ou supérieure à 100 mW.

18. Appareil selon la revendication 16 ou 17, dans lequel le faisceau laser est généré dans une gamme de longueurs d'onde de 400 nm ou plus.

19. Laboratoire sur puce comprenant l'appareil selon les revendications 13 à 18.

20. Bille pour isoler et purifier de l'acide nucléique à partir d'une cellule ou d'un virus, où un matériau représenté par la Formule 1 ci-dessous est lié à la surface de la bille : 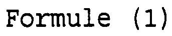 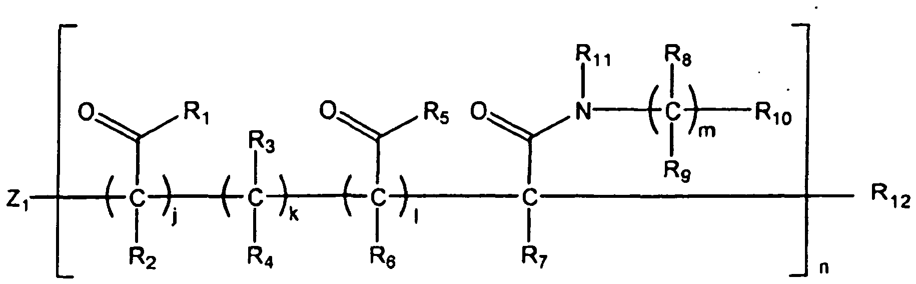 où Z₁, R₁, R₂, R₃, R₄, R₅, R₆, R₇, R₈, R₉, R₁₀, R₁₁, R₁₂, j, k, 1, m et n sont tels que définis dans la revendication 1.

21. Bille selon la revendication 20, où R₁₀ est un groupe pyridinyle ou un groupe imidazolyle.

22. Bille selon la revendication 20 ou 21, où R₁ et R₅ sont chacun un groupe hydroxyle.
